(19) Europäisches Patentamt
European Patent Office
Office européen des brevets

(11) Veröffentlichungsnummer: **0 003 520**
**B1**

# (12) EUROPÄISCHE PATENTSCHRIFT

(45) Veröffentlichungstag der Patentschrift: **15.07.81**

(51) Int. Cl.³: **C 07 D 263/52,** A 01 N 43/76

(21) Anmeldenummer: **79100184.5**

(22) Anmeldetag: **22.01.79**

## (54) Spiro-Derivate von 3-(3,5-Dihalogenphenyl)-oxazolidin-2,4-dionen, Verfahren zu ihrer Herstellung sowie ihre Verwendung als Fungizide.

(30) Priorität: **04.02.78 DE 2804824**

(43) Veröffentlichungstag der Anmeldung: **22.08.79 Patentblatt 79/17**

(45) Bekanntmachung des Hinweises auf die Patenterteilung: **15.07.81 Patentblatt 81/28**

(84) Benannte Vertragsstaaten: **BE CH DE FR GB IT NL**

(56) Entgegenhaltungen:
**GB-A-982 940**

**CHEMICAL SUBSTANCE INDEX, 9th Collective Index, Columbus, Ohio, USA, Seite 26151CS**

**CHEMICAL ABSTRACTS, Vol. 76, Nr. 25, 19, Juni 1972, Zusammenfassung Nr. 149465g, Columbus, Ohio, USA A. FUJINAMI et al.: "Biological activity of cyclic imide compunds. I. Antimicrobial activity of 3-phenyloxazolidine-2,4-diohes and related compounds", Seite 91.**

(73) Patentinhaber: **BAYER AG, Zentralbereich Patente, Marken und Lizenzen, D-5090 Leverkusen 1, Bayerwerk (DE)**

(72) Erfinder: **Knops, Hans-Joachim, Dr., Claudiusweg 3, D-5600 Wuppertal 1 (DE)**
Erfinder: **Heine, Hans-Georg, Dr., Am Heckerhof 14, D-4150 Krefeld (DE)**
Erfinder: **Draber, Wilfried, Dr., In den Birken 81, D-5600 Wuppertal-1 (DE)**
Erfinder: **Brandes, Wilhelm, Dr., Eichendorffstrasse 3, D-5623 Leichlingen (DE)**

EP 0 003 520 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Spiro-Derivate von 3-(3,5-Dihalogenphenyl)-oxazolidin-2,4-dionen, Verfahren zu ihrer Herstellung sowie ihre Vewendung als Fungizide

Die vorliegende Erfindung betrifft neue Spiro-Derivate von 3-(3,5-Dihalogenphenyl)-oxazolidin-2,4-dionen, mehrere Verfahren zu ihrer Herstellung sowie ihre Verwendung als Fungizide.

Es ist bereits bekannt geworden, dass N-(3,5-Dihalogenphenyl)-imide, wie beispielsweise N-(3,5-Dichlorphenyl)-1,2-dimethylcyclopropan-1,2-dicarboximid, gute Mittel zur Bekämpfung von pilzlichen Pflanzenkrankheiten sind (vergleiche DT-OS 2012656). Ebenfalls ist bereits bekannt, dass Thiuramdisulfide, wie beispielsweise Tetramethyl-thiuramdisulfid, gute fungizide Eigenschaften aufweisen (vergleiche US-Patentschrift 1972961). Die Wirkung beider Stoffklassen ist jedoch in bestimmten Indikationsbereichen, insbesondere bei niedrigen Aufwandmengen und -konzentrationen, nicht immer voll befriedigend. Weiterhin ist eine Publikation über antimikrobiell wirksame 3-Phenyloxazolidin-2,4-dione bekannt (Agr. Biol. Chem. 35 (1971), S. 1707-1719), in welcher auch eine Spiro-Verbindung genannt wird (loc. cit. S. 1715); letztere ist jedoch nach Mitteilung der Autoren nur wenig wirksam.

Es wurden nun als neue Verbindungen die Spiro-Derivate von 3-(3,5-Dihalogenphenyl)-oxazolidin-2,4-dionen der Formel

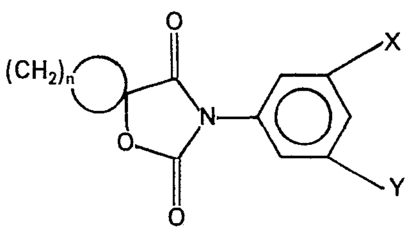

(I)

in welcher

X und Y gleich oder verschieden sind und für Halogen stehen und

n für die ganzen Zahlen 2 oder 3 steht,

gefunden. Sie weisen starke fungizide Eigenschaften auf.

Weiterhin wurde gefunden, dass man die Spiro-Derivate von 3-(3,5-Dihalogenphenyl)-oxazolidin-2,4-dionen der Formel (I) erhält, wenn man α-Hydroxy-cycloalkylcarbonsäuren(ester) der Formel

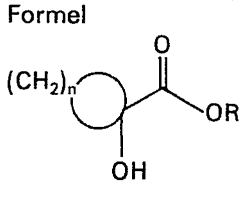

(II)

in welcher

R für Wasserstoff oder Alkyl mit 1 bis 4 Kohlenstoffatomen steht und

n die oben angegebene Bedeutung hat,

a) mit Isocyanaten der Formel

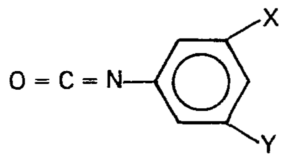

(III)

in welcher

X und Y die oben angegebene Bedeutung haben, gegebenenfalls in Gegenwart einer Base und in Gegenwart eines Verdünnungsmittels umsetzt oder

b) mit Anilinen der Formel

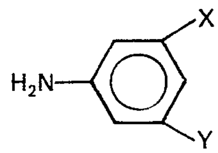

(IV)

in welcher

X und Y die oben angegebene Bedeutung haben, in Gegenwart eines Verdünnungsmittels umsetzt und die entstehenden α-Hydroxy-cycloalkylcarbonsäureamide der Formel

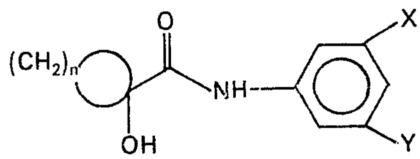

(V)

in welcher

X, Y und n die oben angegebene Bedeutung haben,

mit Phosgen in Gegenwart einer Base cyclisiert.

Überraschenderweise zeigen die erfindungsgemässen Spiro-Derivate von 3-(3,5-Dihalogenphenyl)-oxazolidin-2,4-dionen eine erheblich höhere fungizide Wirkung, insbesondere gegen Botrytis-Arten, als die aus dem Stand der Technik bekannten Verbindungen N-(3,5-Dichlorphenyl)-1,2-dimethyl-cyclopropan-1,2-dicarboximid und Tetramethyl-thiuramdisulfid, welche anerkannt gute Mittel gleicher Wirkungsrichtung sind. Die erfindungsgemässen Wirkstoffe stellen somit eine Bereicherung der Technik dar.

Verwendet man α-Hydroxy-cyclopropancarbonsäure und 3,5-Dichlorphenylisocyanat als Ausgangsstoffe, so kann der Reaktionsablauf durch das folgende Formelschema wiedergegeben werden (Verfahren a):

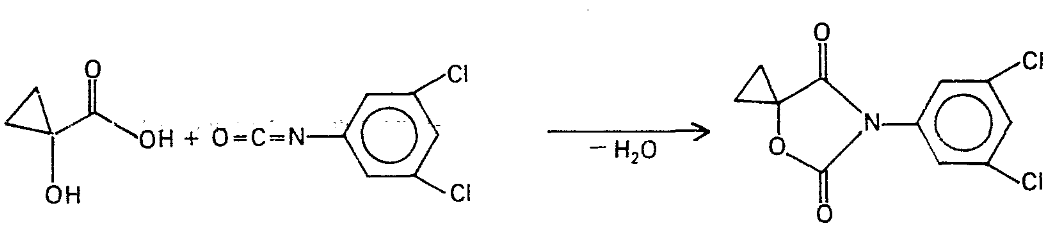

Verwendet man α-Hydroxy-cyclobutancarbonsäure-äthylester und 3,5-Dibromphenylisocyanat als Ausgangsstoffe, so kann der Reaktionsablauf durch das folgende Formelschema wiedergegeben werden (Verfahren a):

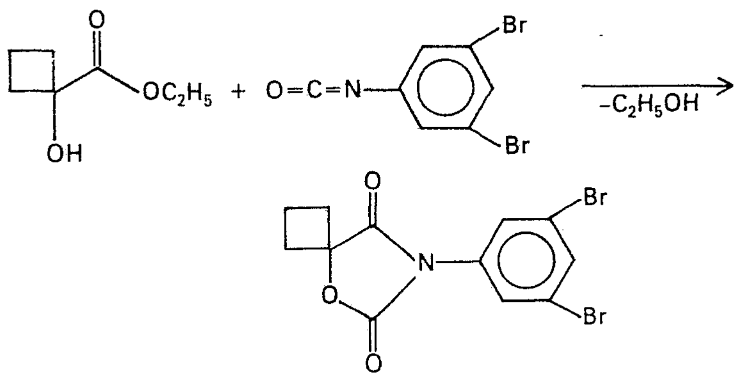


Verwendet man α-Hydroxy-cyclopropancarbonsäure, 3,5-Dichloranilin und Phosgen als Ausgangsstoffe, so kann der Reaktionsablauf durch das folgende Formelschema wiedergegeben werden (Verfahren b):

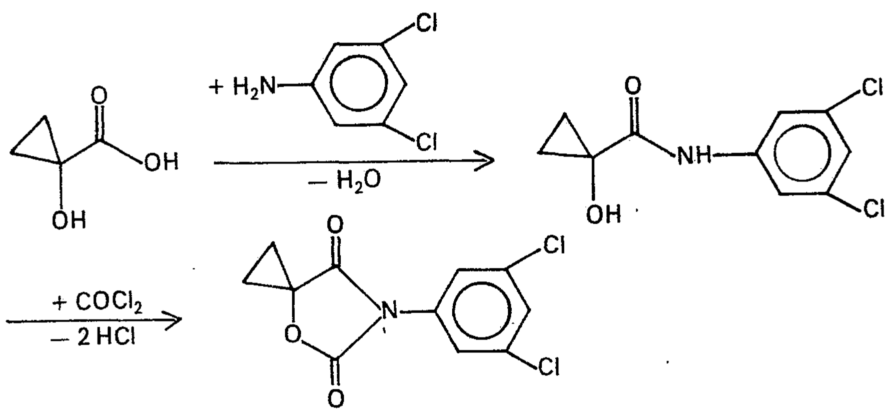


Die als Ausgangsstoffe zu verwendenden α-Hydroxy-cycloalkylcarbonsäuren(ester) sind durch die Formel (II) allgemein definiert. In dieser Formel steht R vorzugsweise für geradkettiges oder verzweigtes Alkyl mit 1 bis 4 Kohlenstoffatomen, wie insbesondere für Methyl oder Äthyl. Der Index n steht vorzugsweise für die ganzen Zahlen 2 oder 3.

Die Ausgangsstoffe der Formel (II) sind bekannt (vergleiche Liebigs Ann. Chem. 1976, 463 sowie Chem. Ber. 55, 2738 (1922)). Als Beispiele seien genannt: α-Hydroxy-cyclopropancarbonsäure, α-Hydroxy-cyclobutancarbonsäure, α-Hydroxy-cyclopropancarbonsäure-methyl- und äthylester, α-Hydroxy-cyclobutancarbonsäure-methyl- und äthylester.

Die α-Hydroxy-cyclopropancarbonsäure kann z.B. in der Weise hergestellt werden, dass man zunächst durch Acyloinkondensation von Bernsteinsäureester in Gegenwart von Trimethylchlorsilan das 1,2-Bis-(trimethylsiloxy)-1-cyclobuten darstellt, dann die erhaltene Verbindung bromiert, wobei man auf dem Wege über das 1,2-Cyclobutandion durch Ringkontraktion die 1-Hydroxy-cyclopropancarbonsäure erhält. Die 1-Hydroxy-cyclobutancarbonsäure kann z.B. dadurch hergestellt werden, dass man Cyclobutancarbonsäure bromiert und die erhaltene 1-Brom-Verbindung anschliessend mit einer wässrigen Kaliumcarbonat-Lösung behandelt.

Die weiterhin als Ausgangsstoffe zu verwendenden Isocyanate sind durch die Formel (III) und die Aniline durch die Formel (IV) allgemein definiert. In diesen Formeln sind X und Y gleich oder verschieden und stehen vorzugsweise für Fluor, Chlor, Brom und Jod.

Die Ausgangsstoffe der Formeln (III) und (IV) sind allgemein bekannte Verbindungen der organischen Chemie.

Für die erfindungsgemässe Umsetzung gemäss Verfahrensvariante (a) kommen als Verdünnungsmittel vorzugsweise inerte organische Lösungsmittel in Frage. Hierzu gehören vorzugsweise aromatische Kohlenwasserstoffe, wie beispielsweise Benzol, Toluol, Xylol oder 1,2-Dichlorbenzol, sowie aliphatische, halogenierte Kohlenwasserstoffe, wie beispielsweise Methylenchlorid, Chloroform oder Tetrachlorkohlenstoff.

Wird die erfindungsgemässe Umsetzung gemäss Verfahrensvariante (a) in Gegenwart einer Base durchgeführt, so können alle üblicherweise verwendbaren organischen und anorganischen Basen eingesetzt werden. Hierzu gehören vorzugsweise tertiäre Amine, wie beispielsweise Triäthylamin oder Pyridin sowie Alkoholate, wie

beispielsweise Kalium- oder Natrium-tert.-butylat.

Die Reaktionstemperaturen können bei der erfindungsgemässen Verfahrensvariante (a) in einem grösseren Bereich variiert werden. Im allgemeinen arbeitet man zwischen 20 und 100°C, vorzugsweise bei der Siedetemperatur des jeweiligen Lösungsmittels.

Bei der Durchführung der erfindungsgemässen Verfahrensvariante (a) arbeitet man vorzugsweise in molaren Mengen. Bei Verwendung einer Base wird diese in äquimolarer Menge eingesetzt, wenn man α-Hydroxycyclopropancarbonsäuren als Ausgangsstoffe verwendet und lediglich in katalytischer Menge, wenn α-Hydroxycyclopropancarbonsäureester eingesetzt werden. Zur Isolierung der Verbindungen der Formel (I) wird das Lösungsmittel abdestilliert und der Rückstand nach üblichen Methoden aufgearbeitet.

Für die erfindungsgemässe Umsetzung gemäss Verfahrensvariante (b) kommen als Verdünnungsmittel vorzugsweise inerte organische Lösungsmittel in Frage. Hierzu gehören vorzugsweise die bei der Verfahrensvariante (a) bereits genannten Solventien.

Als Basen kommen für die erfindungsgemässe Umsetzung gemäss Verfahrensvariante (b) vorzugsweise die bei der Variante (a) bereits genannten Reagenzien in Frage.

Die Reaktionstemperaturen können bei der erfindungsgemässen Verfahrensvariante (b) in einem grösseren Bereich variiert werden. Im allgemeinen arbeitet man zwischen 20 und 100°C, vorzugsweise bei der Siedetemperatur des verwendeten Lösungsmittels.

Bei der Durchführung der erfindungsgemässen Verfahrensvariante (b) arbeitet man vorzugsweise in molaren Mengen. Die als Zwischenprodukt auftretenden α-Hydroxy-cycloalkylcarbonsäureamide der Formel (V) können ohne Isolierung direkt umgesetzt werden. Zur Isolierung der Verbindungen der Formel (I) wird das Lösungsmittel abdestilliert und der Rückstand nach üblichen Methoden aufgearbeitet.

Die erfindungsgemässen Wirkstoffe weisen eine starke fungitoxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes, Chytridiomycetes, Zygomycetes, Deuteromycetes.

Die erfindungsgemässen Wirkstoffe haben ein breites Wirkungsspektrum und können angewandt werden gegen parasitäre Pilze, die oberirdische Pflanzenteile befallen oder die Pflanze vom Boden her angreifen, sowie gegen samenübertragbare Krankheitserreger. Eine besonders gute Wirksamkeit entfalten sie gegen parasitäre Pilze auf oberirdischen Pflanzenteilen.

Als Pflanzenschutzmittel können die erfindungsgemässen Wirkstoffe mit besonders gutem Erfolg zur Bekämpfung von Botrytis-Arten, wie gegen den Erreger des Grauschimmels an Erdbeeren oder Trauben (Botrytis cinerea) verwendet werden.

Als Pflanzenschutzmittel können die erfindungsgemässen Wirkstoffe zur Saatgut- oder Bodenbehandlung und zur Behandlung oberirdischer Pflanzenteile benutzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnussschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweisshydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen in Mischung mit anderen bekannten Wirkstoffen vorliegen, wie Fungiziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertigen Lösungen, Emulsionen, Suspensionen, Pulver, Pasten und Granulate angewendet werden. Die Anwendung geschieht in üblicher Weise, z.B. durch Giessen, Spritzen, Sprühen, Stäuben, Streuen, Trockenbeizen, Feuchtbeizen, Nassbeizen, Schlämmbeizen oder Inkrustieren.

Bei der Verwendung als Blattfungizide können die Wirkstoffkonzentrationen in den Anwendungsformen in einem grösseren Bereich variiert werden. Sie liegen im allgemeinen zwischen 0,1 und 0,00001 Gewichtsprozent. Vorzugsweise zwischen 0,05 und 0,0001%.

Bei der Saatgutbehandlung werden im allgemeinen Wirkstoffmengen von 0,001 bis 50 g je Kilogramm Saatgut, vorzugsweise 0,01 bis 10 g benötigt.

Zur Bodenbehandlung sind Wirkstoffmengen von 1 bis 100 g je cbm Boden, wie insbesondere 10 bis 200 g, erforderlich.

Beispiel A
Botrytis-Test (Bohnen)/protektiv
Lösungsmittel: 4,7 Gewichtsteile Aceton
Dispergiermittel: 0,3 Gewichtsteile Alkyl-arylpolyglykoläther
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man Pflanzen von Phaseolus vulgaris im 2-Blattstadium bis zur Tropfnässe. Nach 24 Stunden werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Grösse der Befallsflecken auf den Blättern bonitiert.

Die erhaltenen Boniturwerte werden auf Prozent Befall umgerechnet. 0% bedeutet keinen Befall, 100% bedeutet, dass der Befallsfleck vollständig ausgebildet ist.

Die Verbindung entsprechend Herstellungsbeispiel (1) zeigt hierbei eine gute, den bekannten Verbindungen N-(3,5-Dichlorphenyl)-1,2-dimethyl-cyclopropan-1,2-dicarboximid und Tetramethyl-thiuramdisulfid überlegene Wirkung.

## HERSTELLUNGSBEISPIELE

Beispiel 1

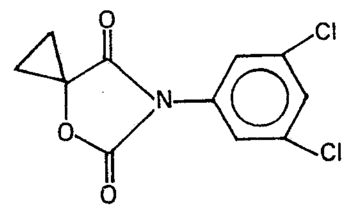

(Verfahrensvariante a)

5,1 g (0,05 Mol) α-Hydroxy-cyclopropancarbonsäure und 5,05 g (0,05 Mol) Triäthylamin werden in 200 ml 1,2-Dichlorbenzol bei ca. 100 °C gelöst. Man tropft eine Lösung von 9,4 g (0,05 Mol) 3,5-Dichlorphenylisocyanat in 300 ml 1,2-Dichlorbenzol hinzu und erhitzt danach 3 Stunden unter Rückfluss am Wasserabscheider. Nach beendeter Wasserabscheidung lässt man abkühlen und engt durch Abdestillieren des Lösungsmittels im Vakuum ein. Der noch warme Rückstand wird mit 100 ml heissem Äthanol versetzt und gut durchgemischt. Während des Abkühlens scheiden sich weisse Kristalle ab, die abgesaugt und aus Äthanol umkristallisiert werden. Man erhält 7 g (51% der Theorie) 1-Oxa-3-azaspiro[4,2]heptan-3-(3,5-dichlorphenyl)-2,4-dion vom Schmelzpunkt 162 °C.

In analoger Weise werden die Verbindungen der folgenden Tabelle 1 erhalten.

Tabelle 1

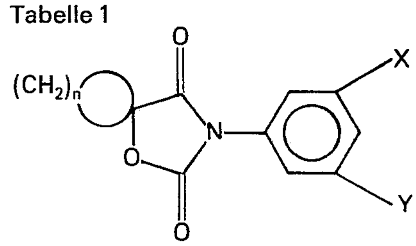

| n | X | Y | |
|---|---|---|---|
| 3 | Cl | Cl | Fp. 148–50 °C |
| 3 | Br | Br | |
| 2 | J | J | |
| 2 | Br | Br | |
| 2 | Cl | Br | |
| 3 | Cl | Br | |

**Patentansprüche:**

1. 3-(3,5-Dihalogenphenyl)-oxazolidin-2,4-dione der Formel

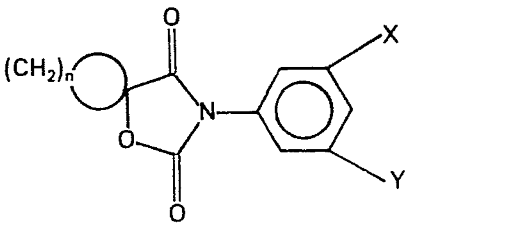

(I)

in welcher
X und Y gleich oder verschieden sind und für Halogen stehen und
n für die ganzen Zahlen 2 oder 3 steht.

2. Verfahren zur Herstellung von 3-(3,5-Dihalogenphenyl)-oxazolidin-2,4-dionen, dadurch gekennzeichnet, dass man α-Hydroxy-cycloalkylcarbonsäuren(ester) der Formel

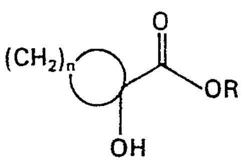

(II)

in welcher
R für Wasserstoff oder Alkyl mit 1 bis 4 Kohlenstoffatomen steht und
n die oben angegebene Bedeutung hat,
a) mit Isocyanaten der Formel

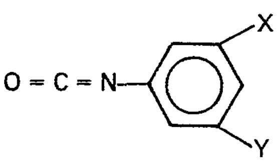

(III)

in welcher
X und Y die oben angegebene Bedeutung haben, gegebenenfalls in Gegenwart einer Base und in Gegenwart eines Verdünnungsmittels umsetzt oder
b) mit Anilinen der Formel

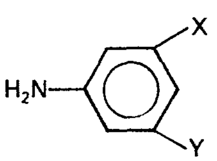

(IV)

in welcher
X und Y die oben angegebene Bedeutung haben, in Gegenwart eines Verdünnungsmittels umsetzt und die entstehenden α-Hydroxy-cycloalkylcarbonsäureamide der Formel

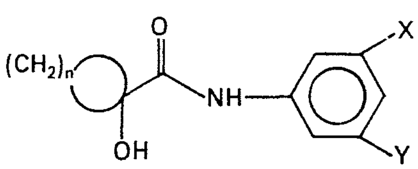

(V)

in welcher
X, Y und n die oben angegebene Bedeutung haben,
mit Phosgen in Gegenwart einer Base cyclisiert.

3. Fungizide Mittel, gekennzeichnet durch einen Gehalt an mindestens einem 3-(3,5-Dihalogenphenyl)-oxazolidin-2,4-dion gemäss Anspruch 1.

4. Verwendung von 3-(3,5-Dihalogenphenyl)-oxazolidin-2,4-dionen gemäss Anspruch 1 zur Bekämpfung von Pilzen.

5. Verfahren zur Herstellung von fungiziden Mitteln, dadurch gekennzeichnet, dass man 3-(3,5-Dihalogenphenyl)-oxazolidin-2,4-dione gemäss Anspruch 1 mit Streckmitteln und/oder oberflächenaktiven Mitteln vermischt.

**Claims**

1. 3-(3,5-Dihalogenophenyl)-oxazolidine-2,4-diones of the formula

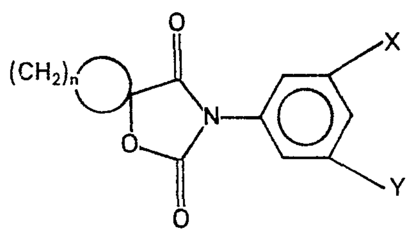

(I)

in which
X and Y are identical or different and represent halogen and
n represents the integers 2 or 3.

2. Process for the preparation of 3-(3,5-dihalogenophenyl)-oxazolidine-2,4-diones, characterised in that α-hydroxy-cycloalkylcarboxylic acids (esters) of the formula

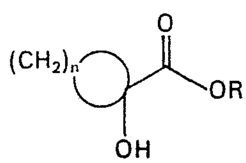

(II)

in which
R represents hydrogen or alkyl with 1 to 4 carbon atoms and
n has the meaning indicated above,
a) are reacted with isocyanates of the formula

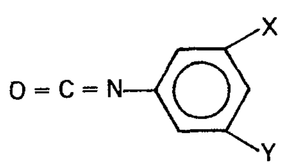

(III)

in which
X and Y have the meaning indicated above,
optionally in the presence of a base and in the presence of a diluent, or
b) are reacted with anilines of the formula

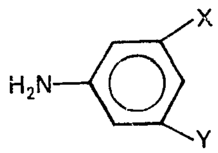

(IV)

in which
X and Y have the meaning indicated above, in the presence of a diluent and the α-hydroxy-cycloalkylcarboxylic acid amides formed, of the formula

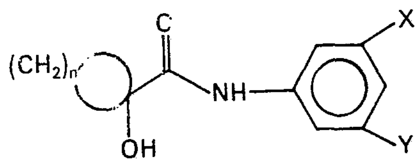

(V)

in which
X, Y and n have the meaning indicated above, are cyclised with phosgene in the presence of a base.

3. Fungicidal agents, characterised in that they contain at least one 3-(3,5-dihalogenophenyl)-oxyzolidine-2,4-dione according to Claim 1.

4. Use of 3-(3,5-dihalogenophenyl)-oxazolidine-2,4-diones according to Claim 1 for combating fungi.

5. Process for the preparation of fungicidal agents, characterised in that 3-(3,5-dihalogenophenyl)-oxazolidine-2,4-diones according to Claim 1 are mixed with extenders and/or surface-active agents.

**Revendications**

1. Des 3-(3,5-dihalogénophényl)-oxazolidine-2,4-diones de formule:

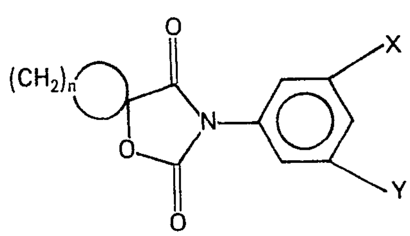

(I)

dans laquelle:
X et Y sont égaux ou différents et représentent un halogène et
n représente les nombres entiers 2 ou 3.

2. Procédé de production de 3-(3,5-dihalogénophényl)-oxazolidine-2,4-diones, caractérisé en ce que des acides ou esters d'acides α-hydroxy-cycloalkylcaboxyliques de formule:

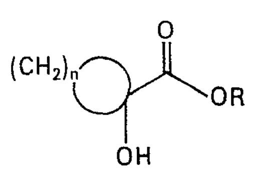

(II)

dans laquelle:
R est de l'hydrogène ou un groupe alkyle ayant 1 à 4 atomes de carbone et
n a la définition donnée ci-dessus

a) sont amenés à réagir avec des isocyanates de formule:

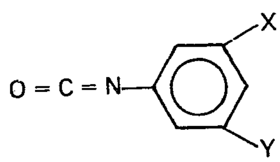

(III)

dans laquelle:
X et Y ont la définition donnée ci-dessus,
le cas échéant en présence d'une base et en présence d'un diluant ou

b) sont amenés à réagir avec des anilines de formule:

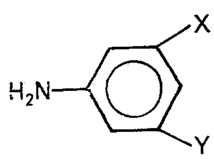

(IV)

dans laquelle:
X et Y ont la définition donnée ci-dessus,
en présence d'un diluant et les amides d'acides α-hydroxy-cycloalkylcarboxyliques produits de formule:

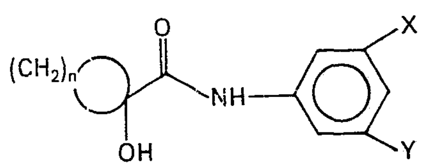

(V)

dans laquelle:
X, Y et n ont la définition donnée ci-dessus,
sont cyclisés avec du phosgène en présence d'une base.

3. Compositions fongicides, caractérisées par une teneur en au moins une 3-(3,5-dihalogénophényl)-oxazolidine-2,4-dione suivant la revendication 1.

4. Utilisation de 3-(3,5-dihalogénophényl)-oxazolidine-2,4-diones suivant la revendication 1, pour la lutte contre des champignons.

5. Procédé de production de compositions fongicides, caractérisé en ce qu'on mélange des 3-(3,5-dihalogénophényl)-oxazolidine-2,4-diones suivant la revendication 1 avec des diluants et/ou des agents tensio-actifs.